# EUROPEAN PATENT APPLICATION

(11) **EP 4 772 178 A1**
(43) Date of publication of application: **08.07.2026**
(21) Application number: 24857635.7
(22) Date of filing: 21.02.2024
(51) Int. Cl.: A61K 31/365, A61K 31/4188, A61P 35/00

(54) **ANTI-TUMOR USE OF ACT001 COMPOSITION**

(30) Priority: 28.08.2023 CN 202311091345
(71) Applicant: Accendatech Co. Ltd., Tianjin 300102 (CN)
(72) Inventor: CAI, Dongpo, Tianjin 300102 (CN); BAO, Shiqi, Tianjin 300102 (CN); WANG, Lei, Tianjin 300102 (CN); QIAO, Di, Tianjin 300102 (CN); QIU, Chuanjiang, Tianjin 300102 (CN)
(74) Representative: Gille Hrabal Partnerschaftsgesellschaft mbB Patentanwälte
(86) International application number: PCT/CN2024/077905
(87) International publication number: WO 2025/044079

(57) **Abstract**

A drug combination composition comprising ACT001 has a synergistic anti-tumor effect in a glioblastoma (GBM) animal model, effectively protects or improves, in the process of a phase II clinical trial of treating a GBM patient who was sensitive to temozolomide (TMZ) but subsequently relapses, basic immune functions of said patient, such as relieving lymphocyte reduction caused by chemotherapy, alleviating myelosuppression of TMZ, and suppressing inhibitory Treg lymphocytes in peripheral blood, and reduces DNA damage repair capability in GBM cancer cells, thereby improving the sensitivity of GBM to TMZ.

## Description

The present application claims priority to the prior application with the application No. 202311091345.8 and entitled "ANTI-TUMOR USE OF ACT001 COMPOSITION" filed with China National Intellectual Property Administration on August 28, 2023, which is incorporated herein by reference in its entirety.

### TECHNICAL FIELD

The present disclosure relates to the technical field of pharmaceuticals and particularly relates to anti-tumor use of an ACT001 composition.

### BACKGROUND

In 2005, the FDA approved TMZ for the treatment of GBM (glioblastoma) (Stupp, et al., 2005). However, the high heterogeneity of cancer and its adaptability during treatment are the primary reasons why nearly all subsequent Phase II and III clinical trials failed in the end. Years of translational and clinical research have shown that to successfully develop a drug for GBM, the following criteria should be met:
1) Early clinical trial data, especially trial data from the small number of patients who experience genuine clinical benefit, are crucial. In the field of GBM drug development, there is a huge gap between preclinical data and clinical reality. For example, clinical trials have confirmed that the combination of ACT001 and pembrolizumab does not yield higher efficacy than ACT001 alone, and also that the preclinical data of combinations of parthenolide and PD-1 antibodies do not correlate with clinical reality.
2) Developing drugs aimed at all GBM subtype patients defies the pathogenesis and biological logic of GBM. Instead, patents and development aimed at specific GBM subsets hold greater promise.
3) Selecting GBM subset patients who match the mechanism of action of a specific drug is just as important as the pharmacological characteristics of the drug itself.
4) The success of tumor-treating fields (TTFs) suggests that on the basis of the existing effective GBM treatment (TMZ), studying mechanisms of drug resistance development will be more conducive to developing new systemic therapies.
5) Given the diversity of GBM tumors, combination therapies and multi-target drugs hold greater promise in the field of GBM drug development.

The expression level of the MGMT gene is one of the determinants that affect the sensitivity of GBM to TMZ. Overexpression can reduce the sensitivity of GBM cells to TMZ by repairing the DNA damage that TMZ causes. MMR (mismatch repair) is an important factor involved in the treatment of GBM with TMZ. The down-regulation of its expression level will affect the sensitivity of GBM to TMZ (McFaline-Figueroa et al., 2015). Factors affecting the TMZ sensitivity of GBM also include other pathways involved in DNA repair. For example, the process of the treatment of GBM with TMZ leading to cancer cell death requires the use of DNA single-strand breaks (SSBs) or double-strand breaks (DSBs) to achieve further killing effects on cancer cells.

Once GBM develops resistance to TMZ, an important unresolved clinical problem is how to restore its sensitivity to TMZ. The inability to obtain NF-κB inhibitors with sufficient safety windows precluded clinical studies on such problems.

### REFERENCES

McFaline-Figueroa, J. L., Braun, C. J., Stanciu, M., Nagel, Z. D., Mazzucato, P., Sangaraju, D., . . . manson, L. D. (2015). Minor changes in expression of the mismatch repair protein MSH2 exert a major impact on glioblastoma response to temozolomide. Cancer Res, 3127-3138.

Stupp, R., Mason, P. W., van den Bent, J. M., Weller, M., Fisher, B., & Taphoom, B. M. (2005). Radiotherapy plus concomitant and adjuvant temozolomide for glioblastoma. N Engl J Med, 352, 987-996.

### SUMMARY

In order to remedy the technical problems described above, the present disclosure provides a pharmaceutical combination composition comprising ACT001, and use of the pharmaceutical combination composition for the manufacturing of a medicament for the treatment and/or prevention of tumors.

The present disclosure provides a pharmaceutical combination composition comprising:
(i) DMAMCL or a pharmaceutically acceptable salt thereof,
   and (ii) an alkylating agent or a pharmaceutically acceptable salt thereof,
   wherein DMAMCL is represented by the following formula:

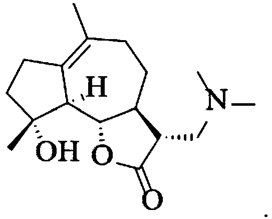

In some embodiments, components (i) and (ii) are in a mass ratio of 0.1-100:1, preferably 1-50:1, and more preferably 5-30:1, e.g., 5-20:1, 5:1, 10:1, 15:1, 20:1, 25:1, or 30:1.

In some embodiments, the pharmaceutically acceptable salt of DMAMCL is DMAMCL fumarate ACT001, wherein ACT001 is represented by the following formula:

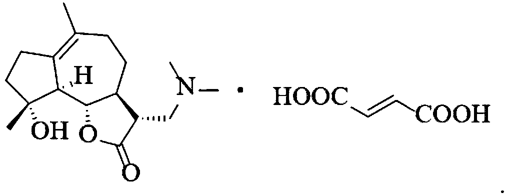

In some embodiments, the alkylating agent is selected from temozolomide (TMZ) and lomustine (CCNU).

In some embodiments, DMAMCL or the pharmaceutically acceptable salt thereof is administered at a dose of 50 mg/kg-500 mg/kg, e.g., 100 mg/kg-300 mg/kg, 150 mg/kg, 200 mg/kg, 250 mg/kg, or 300 mg/kg.

In some embodiments, the alkylating agent or the pharmaceutically acceptable salt thereof is administered at a dose of 1 mg/kg-100 mg/kg, e.g., 90 mg/kg, 80 mg/kg, 70 mg/kg, 60 mg/kg, 50 mg/kg, 40 mg/kg, 30 mg/kg, 20 mg/kg, or 10 mg/kg.

In some embodiments, DMAMCL or the pharmaceutically acceptable salt thereof is administered at a dose of 100 mg-800 mg, 1-3 times daily; preferably at a dose of 300 mg-500 mg, 1-3 times daily; and more preferably at a dose of 400 mg, 2 times daily.

In some embodiments, temozolomide (TMZ) is administered at a dose of 50 mg/m²/day to 400 mg/m²/day, preferably 150 mg/m²/day to 250 mg/m²/day, e.g., 200 mg/m²/day.

In some embodiments, temozolomide (TMZ) is administered on days one to five of each 28-day course of treatment.

In some embodiments, in the combined pharmaceutical composition, DMAMCL or the pharmaceutically acceptable salt thereof and the alkylating agent or the pharmaceutically acceptable salt thereof are each present in the form of a pharmaceutical composition and can be administered simultaneously, sequentially, or at an interval.

In some embodiments, the combined pharmaceutical composition is used for the treatment and/or prevention of a tumor.

In some embodiments, the tumor includes a nervous system tumor and a glioma.

In some embodiments, the glioma is selected from: astrocytoma, brainstem glioma, ependymoma, mixed glioma, oligodendroglioma, and optic glioma.

In some embodiments, the glioma is glioblastoma. Glioblastoma is the most common and aggressive tumor inside the cranium, with a high tendency for recurrence and/or metastasis after surgery, making it difficult to cure.

In some embodiments, the glioblastoma includes primary glioblastoma and secondary glioblastoma. In some embodiments, the tumor is a drug-sensitive tumor, e.g., an alkylating agent-sensitive tumor. In some embodiments, the tumor is alkylating agent-sensitive glioblastoma, e.g., TMZ-sensitive glioblastoma or lomustine-sensitive glioblastoma.

In some embodiments, the tumor is glioblastoma (GBM) that is sensitive to temozolomide (TMZ) after initial diagnosis, glioblastoma (GBM) that is sensitive to temozolomide (TMZ) when initially diagnosed but relapses after treatment, or glioblastoma (GBM) that is sensitive to temozolomide (TMZ) when initially diagnosed but relapses and develops resistance to TMZ after treatment.

In some embodiments, the glioblastoma is methylated MGMT (O6-methylguanine-DNA-methyltransferase) glioblastoma or unmethylated MGMT glioblastoma.

In some embodiments, the combined pharmaceutical composition is capable of protecting a basic immune function in a tumor patient, e.g., reducing chemotherapy-induced lymphopenia, mitigating a myelosuppressive effect of TMZ, and suppressing peripheral blood suppressive Treg lymphocytes. In some embodiments, the combined pharmaceutical composition is capable of improving sensitivity of glioblastoma (GBM) to an alkylating agent (e.g., TMZ).

The present disclosure further provides use of the pharmaceutical combination composition for the manufacturing of a medicament for the treatment and/or prevention of a tumor.

In some embodiments, the tumor includes a nervous system tumor and a glioma.

In some embodiments, the glioma is selected from: astrocytoma, brainstem glioma, ependymoma, mixed glioma, oligodendroglioma, and optic glioma.

In some embodiments, the glioma is glioblastoma.

In some embodiments, the glioblastoma includes primary glioblastoma and secondary glioblastoma. In some embodiments, the tumor is a drug-sensitive tumor, e.g., an alkylating agent-sensitive tumor. In some embodiments, the tumor is alkylating agent-sensitive glioblastoma, e.g., TMZ-sensitive glioblastoma or lomustine-sensitive glioblastoma.

In some embodiments, the tumor is glioblastoma (GBM) that is sensitive to temozolomide (TMZ) after initial diagnosis, glioblastoma (GBM) that is sensitive to temozolomide (TMZ) when initially diagnosed but relapses after treatment, or glioblastoma (GBM) that is sensitive to temozolomide (TMZ) when initially diagnosed but relapses and develops resistance to TMZ after treatment.

In some embodiments, the glioblastoma is methylated MGMT (O6-methylguanine-DNA-methyltransferase) glioblastoma or unmethylated MGMT glioblastoma.

In some embodiments, the combined pharmaceutical composition is capable of protecting a basic immune function in a tumor patient, e.g., reducing chemotherapy-induced lymphopenia, mitigating a myelosuppressive effect of TMZ, and suppressing peripheral blood suppressive Treg lymphocytes.

In some embodiments, the combined pharmaceutical composition is capable of improving sensitivity of glioblastoma (GBM) to an alkylating agent (e.g., TMZ).

The present disclosure further provides a method of treatment and/or prevention of a tumor, and the method comprises administering to a patient a therapeutically effective amount of the pharmaceutical combination composition described above.

In some embodiments, the tumor includes a nervous system tumor and a glioma.

In some embodiments, the glioma is selected from: astrocytoma, brainstem glioma, ependymoma, mixed glioma, oligodendroglioma, and optic glioma.

In some embodiments, the glioma is glioblastoma.

In some embodiments, the glioblastoma includes primary glioblastoma and secondary glioblastoma. In some embodiments, the tumor is a drug-sensitive tumor, e.g., an alkylating agent-sensitive tumor. In some embodiments, the tumor is alkylating agent-sensitive glioblastoma, e.g., TMZ-sensitive glioblastoma or lomustine-sensitive glioblastoma.

In some embodiments, the tumor is glioblastoma (GBM) that is sensitive to temozolomide (TMZ) after initial diagnosis, glioblastoma (GBM) that is sensitive to temozolomide (TMZ) when initially diagnosed but relapses after treatment, or glioblastoma (GBM) that is sensitive to temozolomide (TMZ) when initially diagnosed but relapses and develops resistance to TMZ after treatment.

In some embodiments, the glioblastoma is methylated MGMT (O6-methylguanine-DNA-methyltransferase) glioblastoma or unmethylated MGMT glioblastoma.

In some embodiments, the combined pharmaceutical composition is capable of protecting a basic immune function in a tumor patient, e.g., reducing chemotherapy-induced lymphopenia, mitigating a myelosuppressive effect of TMZ, and suppressing peripheral blood suppressive Treg lymphocytes.

In some embodiments, the combined pharmaceutical composition is capable of improving sensitivity of glioblastoma (GBM) to an alkylating agent (e.g., TMZ).

In some embodiments, the patient is a patient with glioblastoma (GBM) that is sensitive to temozolomide (TMZ) after initial diagnosis, a patient with glioblastoma (GBM) that is sensitive to temozolomide (TMZ) when initially diagnosed but relapses after treatment, or a patient with glioblastoma (GBM) that is sensitive to temozolomide (TMZ) when initially diagnosed but relapses and develops resistance to TMZ after treatment.

The present disclosure further provides a kit for the treatment of a tumor, and the kit comprises the pharmaceutical combination composition described above.

In some embodiments, the tumor includes a nervous system tumor and a glioma.

In some embodiments, the glioma is selected from: astrocytoma, brainstem glioma, ependymoma, mixed glioma, oligodendroglioma, and optic glioma.

In some embodiments, the glioma is glioblastoma.

In some embodiments, the glioblastoma includes primary glioblastoma and secondary glioblastoma. In some embodiments, the tumor is a drug-sensitive tumor, e.g., an alkylating agent-sensitive tumor. In some embodiments, the tumor is alkylating agent-sensitive glioblastoma, e.g., TMZ-sensitive glioblastoma or lomustine-sensitive glioblastoma.

In some embodiments, the tumor is glioblastoma (GBM) that is sensitive to temozolomide (TMZ) after initial diagnosis, glioblastoma (GBM) that is sensitive to temozolomide (TMZ) when initially diagnosed but relapses after treatment, or glioblastoma (GBM) that is sensitive to temozolomide (TMZ) when initially diagnosed but relapses and develops resistance to TMZ after treatment.

In some embodiments, the glioblastoma is methylated MGMT (O6-methylguanine-DNA-methyltransferase) glioblastoma or unmethylated MGMT glioblastoma.

In some embodiments, the combined pharmaceutical composition is capable of protecting a basic immune function in a tumor patient, e.g., reducing chemotherapy-induced lymphopenia, mitigating a myelosuppressive effect of TMZ, and suppressing peripheral blood suppressive Treg lymphocytes.

In some embodiments, the combined pharmaceutical composition is capable of improving sensitivity of glioblastoma (GBM) to an alkylating agent (e.g., TMZ).

### Beneficial Effects

The present disclosure provides a pharmaceutical combination composition comprising ACT001. The pharmaceutical combination composition has a synergistic anti-tumor effect in a glioblastoma animal model; compared to monotherapy, the pharmaceutical combination composition can significantly reduce the volume and mass of a tumor. Moreover, during the treatment of patients with glioblastoma (especially GBM that had previously been sensitive to TMZ but later relapsed) in a Phase II clinical trial, the pharmaceutical combination composition was capable of effectively protecting or improving the basic immune function in the patients, for example, reducing chemotherapy-induced lymphopenia, mitigating the myelosuppressive effect of TMZ, and suppressing peripheral blood suppressive Treg lymphocytes, while reducing the DNA damage repairing ability in GBM cancer cells, thereby improving the sensitivity of GBM to TMZ.

### BRIEF DESCRIPTION OF THE DRAWINGS

FIG. 1 shows the results of the treatment of U118 cells with ACT001 in combination with TMZ - growth curves.
FIG. 2 shows the results of the treatment of U118 cells with ACT001 in combination with TMZ - changes in the weight of tumor tissues.
FIG. 3 shows the specific inhibition ratios of tumor volume and tumor weight for each group.

### DETAILED DESCRIPTION

The technical solutions of the present disclosure are further described in detail below with reference to specific examples. It will be appreciated that the following examples are only illustrative of the present disclosure and should not be construed as limiting the protection scope of the present disclosure. All techniques implemented on the basis of the content described above of the present disclosure fall within the protection scope of the present disclosure.

Unless otherwise stated, the starting materials and reagents used in the following examples are all commercially available products or can be prepared by known methods.

### Example 1

**Model:** human glioma cell line U118MG nude mouse subcutaneous graft tumor model

**Method of establishing subcutaneous preservation U118MG cell model:** After *in vivo* passage to the third generation, when the subcutaneous tumor grew to a diameter of about 1-2 cm, the tumor mass was taken out under sterile conditions and cut into tumor masses of about 1.5 × 1.5 mm. Five-week-old nude mice were taken, and a prepared tumor mass was subcutaneously inoculated on the back of the right forelimb of each mouse. When the tumors grew to an appropriate size, the mice were divided into groups of 10 according to tumor volume and dosed.

### Administration method:

Administration was started on the day of grouping for each treatment group.

ACT001 was dissolved in normal saline, and the solution was administered intragastrically once daily, with a one-day break from administration taken each week.

The contents of temozolomide capsules were taken and weighed, and the dosage level was calculated based on the strength. The contents were suspended in normal saline, and the suspension was administered intragastrically at 1 mg/kg once daily for 7 days.

### Data processing:

Data are expressed using ± s; tumor volume = 1/2ab² (a = long diameter of tumor; b = short diameter of tumor); relative tumor volume (RTV) = Vt/Vo (Vo = tumor volume before administration; Vt = each tumor volume measurement after administration); tumor volume inhibition ratio = (volume of control group - volume of treatment group)/volume of control group × 100%; tumor weight growth inhibition ratio = (tumor weight of control group - tumor weight of treatment group)/tumor weight of control group × 100%. Intergroup statistical analysis was performed using a t-test program in EXCEL software.

The Q value of the combination therapy was calculated: Q = Ea+b/(Ea + Eb - Ea × Eb), where Ea+b is the inhibition ratio of the combination therapy, and Ea and Eb are the inhibition ratios of drug A monotherapy and drug B monotherapy, respectively. This value was used to determine whether the combination therapy had a synergistic effect (Q < 0.85 indicates antagonism, 0.85 ≤ Q < 1.15 indicates addition, and Q ≥ 1.15 indicates synergism).

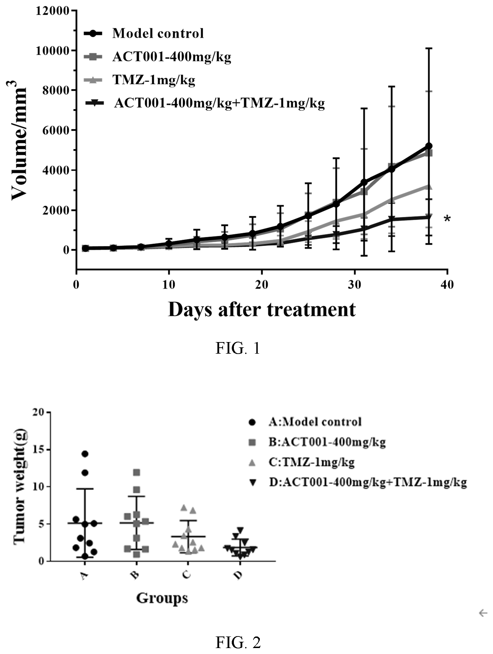

The efficacy of the combination of ACT001 and TMZ was tested in the GBM (U-118) xenograft mouse model constructed above. FIGs. 1, 2, and 3 show tumor volume and tumor weight measurements and specific inhibition ratios for each group in the model, respectively.

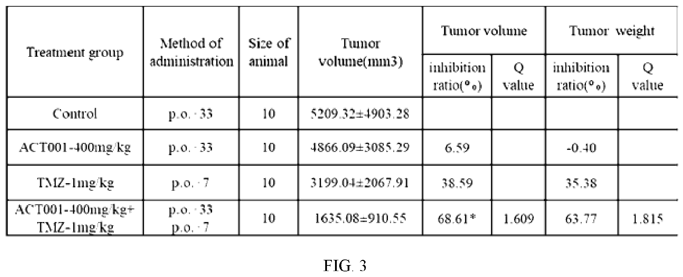

As shown in FIG. 1, at the end of treatment, the tumor volume of the ACT001 + TMZ combination therapy group after treatment was significantly lower than those of the ACT001 monotherapy group and the TMZ monotherapy group; as shown in FIG. 2, the tumor weight of the combination therapy group was significantly lower than those of the ACT001 monotherapy group and the TMZ monotherapy group. Moreover, the Q value ≥ 1.15 also shows that ACT001 and TMZ had an anti-tumor synergistic effect (FIG. 3).

### Example 2

GBM patients who relapsed after standard treatment were randomly assigned to three cohorts of an ACT001-CN-002 Phase II clinical trial:
- **Cohort A:** ACT001 monotherapy (400 mg, BID);
- **Cohort B:** ACT001 and TMZ (temozolomide) were administered in combination at doses of 400mg, BID (ACT001) and 200 mg/m²/day (TMZ, administered on days one to five of each 28-day course of treatment), respectively;
- **Cohort C:** CCNU monotherapy (lomustine, 100-130 mg/m²/dose, once every six weeks, 4 doses at most) or TMZ monotherapy (the dosage level and the route of administration were the same as those for cohort B).

The trial endpoints were post-dose progression-free survival (iRANO criteria), one-year survival rate, and safety assessment: the number of times each safety event occurred (e.g., the incidence of peripheral blood lymphopenia). The exploratory endpoints included changes in the peripheral blood suppressive Treg lymphocyte ratio. The trial results are summarized below (Table 1):

**Table 1. The results of a Phase II clinical trial of the combination of ACT001 and TMZ for the treatment of GBM**

| **Sensitive to first-line TMZ treatment or not** | **MGMT methylated?** | **Patient ID** | **Diagnosis** | **Trial cohort** | | **Best response and time (months)** | | **Grade 3 or higher lymphopenia** | **Peripheral blood Treg ratio (before and after treatment)** |
|---|---|---|---|---|---|---|---|---|---|
| No | No | A1 | GBM | **A** | **ACT001** | PD | | No | 3.2/8.4 |
| No | Yes | A2 | GBM | | **ACT001** | SD | **9** | No | 8.7/6.5 |
| Yes | Not known | A3 | GBM | | **ACT001** | PD | | No | 5.9/6.7 |
| No | No | A4 | GBM | | **ACT001** | SD | **4** | No | 6.9/1.8 |
| No | No | 5A | GBM | | **ACT001** | Not known | | No | Not applicable |
| No | Not known | A6 | GBM | | **ACT001** | PD | | No | Not done |
| No | No | A7 | GBM | | **ACT001** | PD | | Not known | 2.4/9.4 |
| No | No | B1 | GBM | **B** | **ACT001+ TMZ** | PD | | No | 8.6/3.0 |
| No | No | B2 | GBM | | **ACT001+ TMZ** | SD | **3** | No | 11.1/11.5 |
| No | No | B3 | GBM | | **ACT001+ TMZ** | PD | | No | 63/5.4 |
| Yes | Yes | B4 | GBM | | **ACT001+ TMZ** | SD | **≥24** | Yes | 7.4/5.5 |
| Yes | No | B5 | GBM | | **ACT001+ TMZ** | SD | **≥23** | No | 6.0/0.4 |
| No | No | B6 | GBM | | **ACT001+ TMZ** | SD | **4** | No | 5.7/5.3 |
| No | Yes | C1 | GBM | **C** | **CCNU** | SD | **6** | No | 6.6/12.4 |
| Yes | Yes | C2 | GBM | | **TMZ** | SD | **7** | Yes | 4.3/14.3 |
| No | Not known | C4 | GBM | | **CCNU** | Not known | | No | 5.6/7.3 |
| No | No | C5 | GBM | | **CCNU** | Not known | | Yes | 11.4/9.5 |
| No | Yes | C6 | GBM | | **TMZ** | SD | **4** | No | 7.1/7.5 |

| | | | | | | | | | |
|---|---|---|---|---|---|---|---|---|---|
| Note: For patient B4, the tumor size grew after administration, but the clinical condition was stable, and the tumor size gradually decreased later-a decrease of up to 30% compared to the peak value. | | | | | | | | | |

As can be seen from Table 1, in combination therapy group B, both GBM patients B4 and B5 were sensitive to TMZ during first-line treatment. Patient B4, a GBM patient positive for MGMT gene methylation, maintained stable disease, as assessed per the iRANO criteria, for at least 24 months following the combination therapy; in addition, there was a significant increase in lymphocyte count (indicating mitigation of TMZ-induced myelosuppression by the combination therapy), and peripheral blood suppressive Treg lymphocytes were suppressed to some extent. Patient B5, a GBM patient with unmethylated MGMT, maintained stable disease for at least 23 months following the combination therapy, and peripheral blood suppressive Treg lymphocytes were significantly suppressed. By contrast, all the other GBM patients' progression-free survival or stable disease duration did not exceed 9 months.

The use of ACT001 in combination with TMZ enhanced the anti-tumor effect of the individual drug TMZ in recurrent GBM patients with particular clinical backgrounds. In combination therapy group B, both GBM patients B4 and B5 experienced long-term clinical benefit lasting up to nearly two years, which surpassed historical data. Both GBM patients who experienced long-term benefit from the combination therapy had a history of being sensitive to TMZ during first-line treatment; however, the other patients who had a history of being sensitive to TMZ during first-line treatment did not experience significant clinical benefit from ACT001 or TMZ monotherapy, suggesting that the combination therapy had a unique therapeutic effect. Given that B4 and B5 had previously been sensitive to TMZ but later relapsed, such GBM patients represent an ideal model for studying drug sensitization and have direct clinical significance. ACT001 may sensitize GBM to TMZ by mechanisms such as lowering MGMT expression levels in cancer cells of patients who relapse, or inhibiting the DNA double-strand break repairing function of cancer cells. Given that the proportion of MGMT gene methylation could be up to 50% among all cancer patients' tumor samples, the findings of the present disclosure provide, for the first time, an actionable pathway for patient clinical background screening and biomarker exploration, offering guidance for future development of drugs for GBM and other cancers.

The embodiments of the present disclosure have been described above. However, the embodiments described above are not intended to limit the present disclosure. Any modifications, equivalent replacements, improvements, etc., made without departing from the spirit and principles of the present disclosure shall fall within the protection scope of the present disclosure.

## Claims

1. A pharmaceutical combination composition, comprising:
(i) DMAMCL or a pharmaceutically acceptable salt thereof,
and (ii) an alkylating agent or a pharmaceutically acceptable salt thereof,
wherein DMAMCL is represented by the following formula:

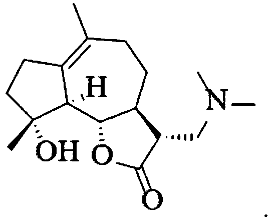

2. The pharmaceutical combination composition as claimed in claim 1, wherein components (i) and (ii) are in a mass ratio of 0.1-100:1, preferably 1-50:1, and more preferably 5-30:1;
and/or the pharmaceutically acceptable salt of DMAMCL is DMAMCL fumarate ACT001, wherein ACT001 is represented by the following formula: 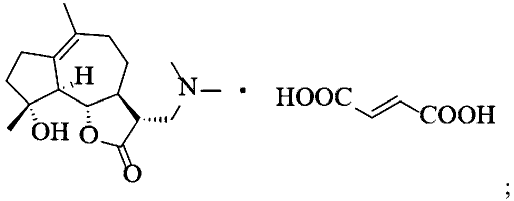
and/or the alkylating agent is selected from temozolomide (TMZ) and lomustine (CCNU);
and/or DMAMCL or the pharmaceutically acceptable salt thereof is administered at a dose of 50 mg/kg-500 mg/kg, e.g., 100 mg/kg-300 mg/kg;
and/or the alkylating agent or the pharmaceutically acceptable salt thereof is administered at a dose of 1 mg/kg-100 mg/kg;
and/or DMAMCL or the pharmaceutically acceptable salt thereof is administered at a dose of 100 mg-800 mg, 1-3 times daily; preferably at a dose of 300 mg-500 mg, 1-3 times daily; and more preferably at a dose of 400 mg, 2 times daily.

3. The pharmaceutical combination composition as claimed in claim 1 or 2, wherein
temozolomide (TMZ) is administered at a dose of 50 mg/m²/day to 400 mg/m²/day, preferably 150 mg/m²/day to 250 mg/m²/day, e.g., 200 mg/m²/day;
and/or temozolomide (TMZ) is administered on days one to five of each 28-day course of treatment;
and/or in the combined pharmaceutical composition, DMAMCL or the pharmaceutically acceptable salt thereof and the alkylating agent or the pharmaceutically acceptable salt thereof are each present in the form of a pharmaceutical composition and can be administered simultaneously, sequentially, or at an interval.

4. Use of the pharmaceutical combination composition as claimed in any one of claims 1-3 for the manufacturing of a medicament for the treatment and/or prevention of a tumor.

5. The use as claimed in claim 4, wherein the tumor includes a nervous system tumor and a glioma;
preferably, the glioma is selected from: astrocytoma, brainstem glioma, ependymoma, mixed glioma, oligodendroglioma, and optic glioma;
preferably, the glioma is glioblastoma;
preferably, the glioblastoma includes primary glioblastoma and secondary glioblastoma;
preferably, the tumor is a drug-sensitive tumor, e.g., an alkylating agent-sensitive tumor;
preferably, the tumor is alkylating agent-sensitive glioblastoma, e.g., TMZ-sensitive glioblastoma or lomustine-sensitive glioblastoma;
preferably, the tumor is glioblastoma (GBM) that is sensitive to temozolomide (TMZ) after initial diagnosis, glioblastoma (GBM) that is sensitive to temozolomide (TMZ) when initially diagnosed but relapses after treatment, or glioblastoma (GBM) that is sensitive to temozolomide (TMZ) when initially diagnosed but relapses and develops resistance to TMZ after treatment;
preferably, the glioblastoma is methylated MGMT (O6-methylguanine-DNA-methyltransferase) glioblastoma or unmethylated MGMT glioblastoma;
preferably, the combined pharmaceutical composition is capable of protecting a basic immune function in a tumor patient, e.g., reducing chemotherapy-induced lymphopenia, mitigating a myelosuppressive effect of TMZ, and suppressing peripheral blood suppressive Treg lymphocytes;
preferably, the combined pharmaceutical composition is capable of improving sensitivity of glioblastoma (GBM) to an alkylating agent (e.g., TMZ).

6. A method of treatment and/or prevention of a tumor, comprising administering to a patient a therapeutically effective amount of the pharmaceutical combination composition as claimed in any one of claims 1-3.

7. The method as claimed in claim 6, wherein the tumor includes a nervous system tumor and a glioma;
preferably, the glioma is selected from: astrocytoma, brainstem glioma, ependymoma, mixed glioma, oligodendroglioma, and optic glioma;
preferably, the glioma is glioblastoma;
preferably, the glioblastoma includes primary glioblastoma and secondary glioblastoma;
preferably, the tumor is a drug-sensitive tumor, e.g., an alkylating agent-sensitive tumor;
preferably, the tumor is alkylating agent-sensitive glioblastoma, e.g., TMZ-sensitive glioblastoma or lomustine-sensitive glioblastoma;
preferably, the tumor is glioblastoma (GBM) that is sensitive to temozolomide (TMZ) after initial diagnosis, glioblastoma (GBM) that is sensitive to temozolomide (TMZ) when initially diagnosed but relapses after treatment, or glioblastoma (GBM) that is sensitive to temozolomide (TMZ) when initially diagnosed but relapses and develops resistance to TMZ after treatment;
preferably, the glioblastoma is methylated MGMT (O6-methylguanine-DNA-methyltransferase) glioblastoma or unmethylated MGMT glioblastoma;
preferably, the combined pharmaceutical composition is capable of protecting a basic immune function in a tumor patient, e.g., reducing chemotherapy-induced lymphopenia, mitigating a myelosuppressive effect of TMZ, and suppressing peripheral blood suppressive Treg lymphocytes;
preferably, the combined pharmaceutical composition is capable of improving sensitivity of glioblastoma (GBM) to an alkylating agent (e.g., TMZ);
preferably, the patient is a patient with glioblastoma (GBM) that is sensitive to temozolomide (TMZ) after initial diagnosis, a patient with glioblastoma (GBM) that is sensitive to temozolomide (TMZ) when initially diagnosed but relapses after treatment, or a patient with glioblastoma (GBM) that is sensitive to temozolomide (TMZ) when initially diagnosed but relapses and develops resistance to TMZ after treatment.

8. A kit for the treatment of a tumor, comprising the pharmaceutical combination composition as claimed in any one of claims 1-3.

9. The kit as claimed in claim 8, wherein the tumor includes a nervous system tumor and a glioma;
preferably, the glioma is selected from: astrocytoma, brainstem glioma, ependymoma, mixed glioma, oligodendroglioma, and optic glioma;
preferably, the glioma is glioblastoma;
preferably, the glioblastoma includes primary glioblastoma and secondary glioblastoma;
preferably, the tumor is a drug-sensitive tumor, e.g., an alkylating agent-sensitive tumor;
preferably, the tumor is alkylating agent-sensitive glioblastoma, e.g., TMZ-sensitive glioblastoma or lomustine-sensitive glioblastoma;
preferably, the tumor is glioblastoma (GBM) that is sensitive to temozolomide (TMZ) after initial diagnosis, glioblastoma (GBM) that is sensitive to temozolomide (TMZ) when initially diagnosed but relapses after treatment, or glioblastoma (GBM) that is sensitive to temozolomide (TMZ) when initially diagnosed but relapses and develops resistance to TMZ after treatment;
preferably, the glioblastoma is methylated MGMT (O6-methylguanine-DNA-methyltransferase) glioblastoma or unmethylated MGMT glioblastoma;
preferably, the combined pharmaceutical composition is capable of protecting a basic immune function in a tumor patient, e.g., reducing chemotherapy-induced lymphopenia, mitigating a myelosuppressive effect of TMZ, and suppressing peripheral blood suppressive Treg lymphocytes;
preferably, the combined pharmaceutical composition is capable of improving sensitivity of glioblastoma (GBM) to an alkylating agent (e.g., TMZ).
